# EUROPÄISCHE PATENTSCHRIFT

(11) **EP 1 189 993 B1**
(45) Veröffentlichungstag und Bekanntmachung des Hinweises auf die Patenterteilung: **18.02.2004**
(21) Anmeldenummer: 00949238.0
(22) Anmeldetag: 01.07.2000
(51) Int. Cl.: C09B 51/00, C09B 1/28, C09B 1/30, C09B 69/00, A61K 7/13

(54) **NEUE FARBSTOFFE UND FÄRBEMITTEL**
NOVEL DYES AND COLORANTS
NOUVELLES TEINTURES ET NOUVEAUX COLORANTS

(30) Priorität: 06.07.1999 DE 19930927
(43) Veröffentlichungstag der Anmeldung: 27.03.2002
(73) Patentinhaber: Henkel Kommanditgesellschaft auf Aktien, 40589 Düsseldorf-Holthausen (DE)
(72) Erfinder: HOLLENBERG, Detlef, D-40699 Erkrath (DE); HÖFFKES, Horst, D-40595 Düsseldorf (US); FOITZIK, Joachim-Kurt, D-64342 Seeheim-Jugenheim (DE); ROSE, David, D-40723 Hilden (DE); NAUMANN, Frank, D-40593 Düsseldorf (DE); WOLFRAM, Leszek, J., Stamfort, CT 06902 (US)
(86) Internationale Anmeldenummer: PCT/EP2000/006159
(87) Internationale Veröffentlichungsnummer: WO 2001/002492

(56) Entgegenhaltungen:
- WO-A-99/20234
- DE-A- 1 569 819
- DE-A- 1 901 500
- DE-A- 2 613 425
- DE-A- 4 016 177
- DE-B- 1 083 459
- FR-A- 2 156 250
- FR-A- 2 282 456
- FR-A- 2 456 764

## Beschreibung

Die Erfindung betrifft neue Farbstoffe und Farbstoffvorprodukte, die insbesondere zum Färben von Keratinfasern geeignet sind, die Verwendung dieser Farbstoffe und Farbstoffvorprodukte sowie diese Farbstoffe und/oder Farbstoffvorprodukte enthaltende Färbemittel.

Im Rahmen der Produkte, die zur kosmetischen Behandlung des menschlichen Körpers bereitgestellt werden, spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab. so sind im Bereich der Haarfärbung im wesentlichen drei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Für temporäre Färbungen werden üblicherweise Färbe- oder Tönungsmittel verwendet. die als färbende Komponente sogenannte Direktzieher enthalten. Hierbei handelt es sich um Farbstoffmoleküle. die direkt auf das Haar aufziehen und keinen oxidativen Prozeß zur Ausbildung der Farbe mehr benötigen. Zu diesen Farbstoffen gehört beispielsweise das bereits aus dem Altertum zur Färbung von Körper und Haaren bekannte Henna.

Weiterhin hat in jüngster Zeit ein neuartiges Färbeverfahren große Beachtung gefunden. Bei diesem Verfahren werden Vorstufen des natürlichen Haarfarbstoffes Melanin. insbesondere Derivate des Indols oder Indolins, auf das Haar aufgebracht und bilden im Rahmen oxidativer Prozesse im Haar praktisch naturidentische Farbstoffe aus. Ein solches Verfahren mit 5,6-Dihydroxyindolinen als Farbstoffvorprodukte wurde in der EP-B1-530 229 beschrieben. Bei, insbesondere mehrfacher, Anwendung von Mitteln mit 5,6-Dihydroxyindolin ist es möglich, Personen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß.

Zwar ist es prinzipiell möglich, Färbemittel mit lediglich einem Farbstoff oder einer Farbstoffvorstufe zu formulieren. Mit Ausnahme weniger Produkte, die z. B. Vorläufer des Melanins enthalten, haben solche Färbemittel in der Praxis aber nur geringe Bedeutung erlangt.

Kommerzielle Haarfärbeprodukte enthalten vielmehr üblicherweise eine Mischung von etwa 3 bis 8 unterschiedlichen Farbstoffen und/oder Farbstoffvorprodukten. Die einzelnen Farbstoffe weisen aber in der Regel unterschiedliche Aufziehvermögen, Licht-, Schweiß-, Reib- und Waschechtheiten auf, die zudem noch stark von den strukturellen Eigenschaften und dem Pflegezustand des Haares abhängig sein können. Diese Unterschiede sind vor allem dann ausgeprägt, wenn, wie für viele Nuancen bisher unverzichtbar, direktziehende Farbstoffe zur Einstellung der Nuance in Oxidationsfärbemitteln eingesetzt werden.

Es bedarf daher bei der Entwicklung neuer Haarfärbemittel häufig umfangreicher Versuche, nicht nur um bestimmte Nuancen zu erzielen, sondern vor allem um sicherzustellen, daß die Färbung über den gewünschten Zeitraum sowohl in der Nuance als auch in der Intensität stabil ist.

Es wurde nunmehr überraschenderweise gefunden, daß viele der oben genannten Probleme ganz oder zumindest teilweise vermieden werden können, wenn Substanzen eingesetzt werden. die sowohl über die Eigenschaften eines direktziehenden Farbstoffes als auch über die Eigenschaften eines Oxidationsfarbstoffvorproduktes, eines Vorläufers des Melanins oder eines weiteren direktziehenden Farbstoffes verfügen. Insbesondere hat sich gezeigt, daß die Farbstoffe über ein sehr gutes, mit bekannten Haarfarbstoffen bzw. Haarfarbstoffvorprodukten vergleichbares Aufziehvermögen auf das Haar verfügen und zu brillanten, intensiven Haarfärbüngen führen. Aufgrund der molekularen Verknüpfung läßt sich so das Problem unterschiedlicher Echtheitseigenschaften der beiden Farbstoffe bzw. Farbstoffvorprodukte in vielen Fällen weitgehend überwinden.

Solche Substanzen, die im weiteren als "Hybridfarbstoffe" bezeichnet werden, sind neu.

Ein erster Gegenstand der vorliegenden Erfindung sind daher Hybridfarbstoffe, insbesondere zum Färben keratinischer Fasern, der Struktur (I),

X - S - Y (I)

in der
X steht für eine Gruppe, die abgeleitet ist von einem direktziehenden Farbstoff,
Y steht für eine Gruppe, die abgeleitet ist von
- einem Oxidationsfarbstoffvorprodukt vom Kuppler- oder Entwicklertyp,
- einem Derivat des Indols oder Indolins als Vorläufer des Melanins oder
S steht für eine direkte Bindung oder eine Spacer-Gruppe.

Aus den deutschen Offenlegungsschriften 15 69 819 und 15 69 820 sind direktziehende Nitrofarbstoffe bekannt, die sich formal aus 2 aromatischen Systemen zusammensetzen, die über eine Alkylen- oder Hydroxyalkylen-Brücke verknüpft sind. Diese Farbstoffe weisen gegenüber den beiden Ausgangsverbindungen deutlich veränderte Färbeeigenschaften auf. die im wesentlichen in der größeren Molmasse begründet sind. Ein Hinweis auf die Lehre der vorliegenden Erfindung kann diesen Druckschriften allerdings nicht entnommen werden.

Die Verbindungen gemäß Struktur (I) sind mit üblichen Syntheseverfahren der organischen Chemie zugänglich. In diesem Zusammenhang wird ausdrücklich auf die unten genannten Synthesebeispiele verwiesen.

Wie bereits oben ausgeführt, bilden die Strukturprinzipien bekannter Farbstoffklassen die Basis für die neu entwickelten Hybridfarbstoffe.

Die Gruppe X leitet sich von Verbindungen ab, die der Klasse der direktziehenden Farbstoffe zugeordnet werden. Bevorzugte Klassen von direktziehenden Farbstoffen, von denen sich die Gruppe X ableitet, sind:
- 2-Nitro-1,4-diaminobenzol und seine Derivate der allgemeinen Formel

   R¹R²N-C₆H₃(NO₂)-NR³R⁴,

   in der die Gruppen R¹ bis R⁴ unabhängig voneinander insbesondere stehen für Wasserstoff, eine C₁₋₄-Alkylgruppe, eine C₁₋₄-Hydroxyalkylgruppe, eine C₂₋₄-Dihydroxyalkylgruppe, eine C₁₋₄-Aminoalkylgruppe, eine, gegebenenfalls substituierte, Phenylgruppe oder eine C₃₋₈-Cycloalkylgruppe.
   Besonders bevorzugte Vertreter dieser Farbstoffklasse sind die unter den INCI-Bezeichnungen bekannten Verbindungen HC Red No.3, HC Blue No.2, HC Blue No.12 und HC Violet No.1 sowie 1,4-Bis-(2'-hydroxyethyl)amino-2-nitrobenzol und 4-Amino-2-nitro-diphenylamin-2'-carbonsäure und weiterhin das 2-Nitro-1,4-Diaminobenzol.
- Derivate des 4-Nitro-2-aminophenols
   Besonders bevorzugte Vertreter sind Pikraminsäure und ihre Salze, insbesondere das Natriumsalz, 2-Amino-6-chlor-4-nitrophenol und 2-Chlor-6-ethylamino-4-nitrophenol.
- Derivate des 2-Amino-5-nitrophenols
   Ein besonders bevorzugter Vertreter ist die unter der INCI-Bezeichnung HC Violet No.4 bekannte Verbindung.
- Derivate des 2-Nitro-4-aminophenols
   Besonders bevorzugte Vertreter sind die unter der INCI-Bezeichnung HC Red BN bekannte Verbindung sowie 4-(2'Hydroxyethyl)-amino-2-nitrophenol.
- Derivate des 2-Nitro-anilins
   Besonders bevorzugte Vertreter sind die unter den INCI-Bezeichnungen HC Yellow No.2 und HC Yellow No.6 bekannten Verbindungen sowie 4-Ethylamino-3-nitrobenzoesäure und N-Hydroxyethyl-2-nitro-4-methylanilin und weiterhin das 2,4-Dinitro-anilin.
- Derivate des Chinoxalins
   Ein besonders bevorzugter Vertreter ist das 6-Nitro-1,2,3,4-tetrahydrochinoxalin.
- Derivate des Anthrachinons
   Besonders bevorzugte Vertreter sind die unter den INCI-Bezeichnungen Disperse Blue 3, Disperse Violet 1 und Disperse Violet 4 (CI 61105) bekannten Verbindungen und weiterhin das 1-Amino-anthrachinon und die 1,4-Diaminoanthrachinon-3-sulfonsäure.
- Derivate des Naphthochinons
   Ein besonders bevorzugter Vertreter ist die unter der INCI-Bezeichnung Basic Blue 99
   bekannte Verbindung.
- Azofarbstoffe
   Besonders bevorzugte Vertreter sind die unter den INCI-Bezeichnungen Basic Yellow 57 (CI 12719), Disperse Orange 3 (CI 11005), Basic Red 76 (CI 12245), Disperse Black 9, Basic Brown 16 (CI 12250) und Basic Brown 17 (CI 12251) bekannten Verbindungen.

Bezüglich weiterer Direktzieher, von denen sich die Gruppe X ableiten kann, wird weiterhin ausdrücklich auf die bekannten Monographien, wie z. B. Ch. Zviak, The Science of Hair Care, Kapitel 7 (Seiten 248-250), erschienen als Band 7 der Reihe "Dermatology" (Hrg.: Ch. Culnan und H. Maibach), Verlag Marcel Dekker Inc., New York, Basel, 1986. sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel. Reformwaren und Körperpflegemittel e.V., Mannheim. Bezug genommen. Selbstverständlich können sich die Gruppen X auch von in der Natur vorkommenden direktziehenden Farbstoffe wie beispielsweise Henna rot, Henna neutral, Kamillenblüte. Sandelholz, schwarzem Tee, Faulbaumrinde, Salbei, Blauholz, Krappwurzel, Catechu, Sedre und Alkannawurzel ableiten.

Die Gruppe Y steht für eine Gruppe, die abgeleitet ist von
- einem Oxidationsfarbstoffvorprodukt vom Kuppler- oder Entwicklertyp,
- einem Derivat des Indols oder Indolins als Vorläufer des Melanins oder
- einem direktziehenden Farbstoff.

Gemäß einer ersten, bevorzugten, Ausführungsform der vorliegenden Erfindung leitet sich die Gruppe Y ab von einem Oxidationsfarbstoffvorprodukt vom Kupplertyp.

Bevorzugte Klassen von Oxidationsfarbstoffvorprodukten vom Kupplertyp, von denen sich die Gruppe Y ableiten kann, sind
- 3-Aminophenol und dessen Derivate
   Bevorzugte Vertreter sind 5-Amino-2-methylphenol, 5-(3-Hydroxypropylamino)-2-methylphenol, 3-Amino-2-chlor-6-methylphenol, 2-Hydroxy-4-aminophenoxy-ethanol, 3-Amino-6-methoxy-2-methylaminophenol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-Dimethylamino-phenol, 3-Diethylamino-phenol, N-Cyclopentyl-3-aminophenol, 1,3-Dihydroxy-5-(methyl-amino)-benzol, 3-Ethylamino-4-methylphenol und 2,4-Dichlor-3-aminophenol.
- 2-Aminophenol und dessen Derivate
- 1.3-Diaminobenzol und dessen Derivate
   Bevorzugte Vertreter sind 2,4-Diamino-phenoxyethanol, 1.3-Bis-(2,4-diaminophenoxy)-propan. 1-Methoxy-2-amino-4-(2'-hydroxyethylamino)benzol, 1,3-Bis-(2,4-diaminophenyl)-propan. 2,6-Bis-(2-hydroxyethylamino)-1-methylbenzol, 1-Amino-3-bis-(2'-hydroxyethyl)-aminobenzol, 1,2-Bis-(2,4-diaminophenoxy)-benzol und 1,3-Bis-(2,4-diaminophenoxy)-benzol.
- 1,2-Diaminobenzol und dessen Derivate
   Bevorzugte Vertreter sind 3,4-Diaminobenzoesäure und 2,3-Diamino-1-methyl-benzol.
- Di- und Trihydroxybenzole und deren Derivate
   Bevorzugte Vertreter sind Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol sowie weiterhin Resorcindimethylether.
- Pyridinderivate
   Bevorzugte Vertreter sind 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-3,4-diaminopyridin, 2,6-Dihydroxy-4-methylpyridin, 2,6-Diaminopyridin, 2,3-Diamino-6-methoxypyridin und 3,5-Diamino-2,6-dimethoxypyridin.
- Naphthalinderivate
   Bevorzugte Vertreter sind 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin sowie weiterhin 1-Aminonaphthalin.
- Morpholinderivate
   Bevorzugte Vertreter sind 6-Hydroxybenzomorpholin und 6-Aminobenzomorpholin.
- Chinoxalinderivate
   Ein bevorzugter Vertreter ist 6-Methyl-1,2,3,4-tetrahydrochinoxalin.
- Pyrazolderivate
   Ein bevorzugter Vertreter ist 1-Phenyl-3-methylpyrazol-5-on.
- Indolderivate
   Bevorzugte Vertreter sind 4-Hydroxyindol, 6-Hydroxyindol und 7-Hydroxyindol.
- Methylendioxybenzolderivate
   Bevorzugte Vertreter sind 3,4-Methylendioxyphenol, 1-Amino-3,4-methylendioxybenzol und 1-(2'-Hydroxyethyl)-amino-3,4-methylendioxybenzol.
- Heterocyclische Verbindungen, wie sie in den Offenlegungsschriften WO 97/35550. WO 97/35552, WO 97/35553, WO 98/08485 und WO 98/08486, auf die ausdrücklich Bezug genommen wird, offenbart sind.

Bezüglich weiterer Oxidationsfarbstoffvorprodukte vom Kupplertyp, von denen sich die Gruppe Y ableiten kann, wird weiterhin ausdrücklich auf die bekannten Monographien, z. B. Ch. Zviak, The Science of Hair Care, Kapitel 8 (Seiten 264-267), erschienen als Band 7 der Reihe "Dermatology" (Hrg.: Ch., Culnan und H. Maibach), Verlag Marcel Dekker Inc., New York, Basel. 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Gemäß einer zweiten, ebenfalls bevorzugten, Ausführungsform der vorliegenden Erfindung leitet sich die Gruppe Y ab von einem Oxidationsfarbstoffvorprodukt vom Entwicklertyp.

Bevorzugte Klassen von Oxidationsfarbstoffvorprodukten vom Entwicklertyp, von denen sich die Gruppe Y ableiten kann, sind
- 1,4-Diaminobenzol und dessen Derivate
   Bevorzugte Vertreter sind p-Phenylendiamin, p-Toluylendiamin, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1,3-Bis(N(2-hydroxyethyl)-N(4-aminophenylamino))-2-propanol, 1,10-Bis-(2,5-diaminophenyl)-1,4,7,10-tetraoxadecan und 1,4-Bis-(4-aminophenyl)-diazacycloheptan sowie entsprechende Verbindungen mit einem oder mehreren Halogenatomen, insbesondere Chlor und Fluor, am Benzolring.
- 1,2-Diaminobenzol und dessen Derivate
- 4-Aminophenol und dessen Derivate
   Bevorzugte Vertreter sind p-Aminophenol, 2-Chlor-4-aminophenol, 4-Amino-3-methylphenol, 2-Hydroxyethylamino-4-amino-phenol. 4,4'-Diaminodiphenylamin. 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 4-Amino-2-(2-hydroxyethoxy)-phenol.
- 2-Aminophenol und dessen Derivate
   Ein bevorzugter Vertreter ist o-Aminophenol.
- Diaminopyridinderivate
- heterocyclische Hydrazone
- 4-Aminopyrazolderivate
   Bevorzugte Vertreter sind 1-Phenyl-3-carboxyamido-4-amino-pyrazolon-5 sowie 4,5-Diaminopyrazol-Derivate nach EP 0 740 931 bzw. WO 94/08970 wie z. B. 4,5-Diamino-1 -(2'-hydroxyethyl)-pyrazol.
- Pyrimidin-Derivate
   Bevorzugte Vertreter sind 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diamino-pyrimidin und 2-Dimethylamino-4,5,6-triaminopyrimidin.

Bezüglich weiterer Oxidationsfarbstoffvorprodukte vom Entwicklertyp, von denen sich die Gruppe Y ableiten kann, wird weiterhin ausdrücklich auf die bekannten Monographien,
z. B. Ch. Zviak, The Science of Hair Care, Kapitel 8 (Seiten 264-267), erschienen als Band 7 der Reihe "Dermatology" (Hrg.: Ch., Culnan und H. Maibach), Verlag Marcel Dekker Inc.. New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Gemäß einer dritten, bevorzugten, Ausführungsform der vorliegenden Erfindung leitet sich die Gruppe Y ab von einem Vorläufer des Melanins, ausgewählt aus den Derivaten des Indols und Indolins. Im Rahmen der vorliegenden Erfindung werden unter "Vorläufer des Melanins" solche Derivate des Indol und des Indolins verstanden, die im Rahmen eines oxidativen Prozesses in der Lage sind. Melaninfarbstoffe oder entsprechende Melaninfarbstoff-Derivate auszubilden.

Erfindungsgemäß bevorzugt leiten sich die Gruppen Y im Rahmen dieser Ausführungsform von Indolen und Indolinen ab, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Indole und Indoline mit zwei dieser Gruppen, insbesondere zwei Hydroxygruppen, von denen eine oder beide verethert oder verestert sein können, sind besonders bevorzugt.

Erfindungsgemäß besonders bevorzugte Gruppen Y leiten sich von Derivaten des Indolins, wie dem 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 5-Hydroxyindolin, 6-Hydroxyindolin, 5-Aminoindolin, 6-Aminoindolin und 4-Aminoindolin ab.

Ganz besonders bevorzugt sind Derivate des 5,6-Dihydroxyindolins der Formel (IIa), 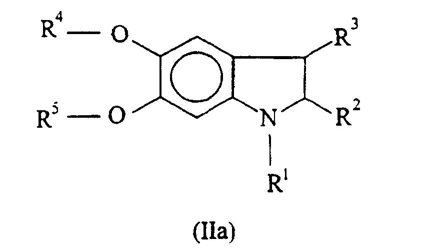 in der unabhängig voneinander
- R¹: steht für Wasserstoff, eine C1-C4-Alkylgruppe, eine C1-C4-Hydroxyalkylgruppe oder eine C3-C6-Cycloalkylgruppe,
- R²: steht für Wasserstoff oder eine -COOH-Gruppe, wobei die -COOH-Gruppe auch als Salz mit einem physiologisch verträglichen Kation vorliegen kann,
- R³: steht für Wasserstoff oder eine C 1-C4-Alkylgruppe.
- R⁴: steht für Wasserstoff, eine C1-C4-Alkylgruppe oder eine Gruppe -CO-R⁶, in der R⁶ steht für eine C1-C4-Alkylgruppe oder eine gegebenenfalls substituierte Phenylgruppe, und
- R⁵: steht für eine der unter R⁴ genannten Gruppen.

Erfindungsgemäß bevorzugte Vertreter sind 5,6-Dihydroxyindolin. N-Methyl-5,6-dihydroxyindolin, N-Ethyl-5,6-dihydroxyindolin, N-Propyl-5,6-dihydroxyindolin, N-Butyl-5,6-dihydroxyindolin. Der Grundkörper, das 5,6-Dihydroxyindolin, ist ganz besonders bevorzugt.

Erfindungsgemäß bevorzugte Indole, von denen sich die Gruppe Y ableitet, sind 5.6-Dihydroxyindol, N-Methyl-5,6-dihydroxyindol, N-Ethyl-5,6-dihydroxyindol, N-Propyl-5,6-dihydroxyindol. N-Butyl-5,6-dihydroxyindol, 5,6-Dihydroxyindol-2-carbonsäure. 5-Hydroxyindol. 6-Hydroxyindol, 5-Aminoindol. 6-Aminoindol und 4-Aminoindol.

Besonders bevorzugt sind Derivate des 5,6-Dihydroxyindols der Formel (IIb), 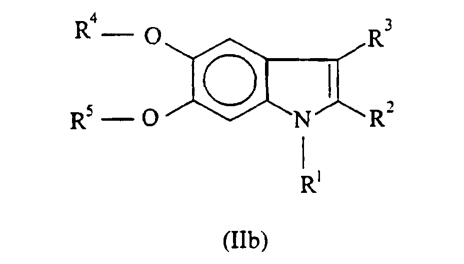 in der unabhängig voneinander
- R¹: steht für Wasserstoff, eine C1-C4-Alkylgruppe, eine C1-C4-Hydroxyalkylgruppe oder eine C3-C6-Cycloalkylgruppe,
- R²: steht für Wasserstoff oder eine -COOH-Gruppe, wobei die -COOH-Gruppe auch als Salz mit einem physiologisch verträglichen Kation vorliegen kann,
- R³: steht für Wasserstoff, eine C1-C4-Alkylgruppe oder eine Gruppe -CH₂-NR⁷R⁸, in der R⁷ und R⁸ unabhängig voneinander stehen für Wasserstoff oder eine C1-C4-Alkylgruppe.
- R⁴: steht für Wasserstoff, eine C1-C4-Alkylgruppe oder eine Gruppe -CO-R⁶, in der R⁶ steht für eine C1-C4-Alkylgruppe oder eine gegebenenfalls substituierte Phenylgruppe, und
- R⁵: steht für eine der unter R⁴ genannten Gruppen.

Erfindungsgemäß bevorzugte Vertreter sind das 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol. Der Grundkörper, das 5,6-Dihydroxyindol, ist ganz besonders bevorzugt.

Wenngleich erfindungsgemäß innerhalb der definierten Gruppe der Hybridfarbstoffe der Struktur (I) solche Verbindungen nicht ausgeschlossen werden sollen, bei denen die Gruppen X und Y identisch sind, so weisen die Verbindungen gemäß dieser Ausführungsform üblicherweise unterschiedliche Gruppen X und Y auf. Insbesondere unterscheiden sich die Gruppen X und Y hinsichtlich ihres Lichtabsorptionsvermögens dahingehend, daß sich die Wellenlängen der Absorptionsmaxima der beiden Gruppen um mindestens 30 nm, bevorzugt um mindestens 50 nm und besonders bevorzugt um mindestens 80 nm, unterscheiden.

Gemäß einer ersten Ausführungsform der Erfindung steht die Gruppe S in der Strukturformel (I) für eine direkte Bindung.

In diesen Fällen wird in den meisten Fällen eine Wechselwirkung der π-Elektronen-Systeme der Gruppen X und Y auftreten, so daß in der Regel das Lichtabsorptionsverhalten des Hybridfarbstoffes sich von dem der Gruppen X und Y deutlich unterscheidet. Dadurch wird auf dem Haar ein signifikant veränderter Farbton erzielt im Vergleich zu Ausfärbungen, die mit einer Mischung entsprechender Farbstoffe durchgeführt werden. die den Gruppen X und Y entsprechen.

Ein Kemgedanke der vorliegenden Erfindung ist jedoch, die bei der Verwendung komplexer Farbstoffmischungen in vielen Bereichen, z. B. hinsichtlich Aufziehvermögen und Waschechtheit, auftretenden Probleme zu beheben, ohne den Farbton und die Nuance zu verändern.

Es ist daher in der Regel erfmdungsgemäß bevorzugt, daß S für eine Spacer-Gruppe steht, über die keine Wechselwirkung der π-Elektronensysteme der Gruppen X und Y stattfindet. Bevorzugt enthält daher S mindestens ein Kohlenstoffatom mit sp³-Hybridisierung auf der direkten Verbindungslinie zwischen den Gruppen X und Y.

Bevorzugte Spacergruppen S sind
- Alkylengruppen der allgemeinen Formel -CₙH₂ₙ-, insbesondere -(CH₂)ₙ-, in denen n für eine ganze Zahl, insbesondere eine Zahl von 1 bis 8 und ganz besonders bevorzugt für eine Zahl von 1 bis 4, steht.
   Erfindungsgemäß bevorzugte Alkylengruppen sind die Methylen-, 1,2-Ethylen- und 1,3-Propylen-Gruppe,
- Cycloaliphatische Gruppen wie Cyclopentyl-, Cyclohexyl- und Cycloheptylgruppen,
- Mono- und Polyhydroxyalkylengruppen der allgemeinen Formel -CₙH₂ₙ₋ₓ(OH)ₓ, in denen n für eine ganze Zahl, insbesondere eine Zahl von 1 bis 8 und ganz besonders bevorzugt für eine Zahl von 1 bis 4, steht und x für eine ganze Zahl, insbesondere für eine Zahl von 1 bis 3, steht.
   Bevorzugte Hydroxyalkylengruppen sind die Hydroxymethylen-, die Hydroxy-1,2-ethylen-, die 2-Hydroxy-1,3-Propylen, die 2,3-Dihydroxy-1,3-propylen- und die 2,3-1,4-Dihydroxybutylen-Gruppe,
- gegebenenfalls an den Alkylketten substituierte Dialkylenaminogruppen, insbesondere solche der allgemeinen Formel -(CH₂)ₙ-N(Z)-(CH₂)ₘ-,
   in der n und m unabhängig voneinander für eine ganze Zahl von 1 bis 8, insbesondere von 1 bis 4, stehen können, jedoch bevorzugt für die gleiche Zahl stehen, und Z für Wasserstoff, eine C₁₋₈-, insbesondere C₁₋₄-,Alkylgruppe, eine C₁₋₈-, insbesondere C₁₋₄-, Mono-hydroxyalkylgruppe, eine C₂₋₈-, insbesondere C₂₋₄-, Dihydroxyalkylgruppe oder eine eine C₃₋₈-, insbesondere C₃₋₄-, Trihydroxyalkylgruppe steht,
   sowie solche der allgemeinen Formel 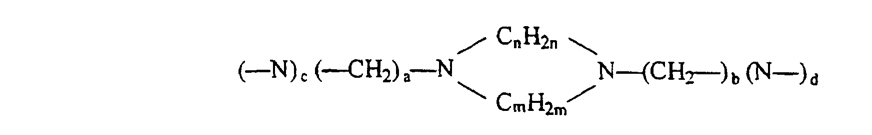 in der a und b unabhängig voneinander für ganze Zahlen von 0 bis 4 und c und d für 0 oder 1 stehen mit der Maßgabe, daß c = 0 ist, wenn a = 0 ist und d = 0 ist, wenn b = 0 ist, n für eine ganze Zahl von 1 bis 5 und m für eine ganze Zahl von 1 bis 3 steht mit der Maßgabe, daß die Summe n+m 3 bis 8 ist. Besonders bevorzugt ist die 1,4-Piperazinogruppe.
- gegebenenfalls an den Alkylketten substituierte Trialkylen-diaminogruppen. insbesondere solche der allgemeinen Formel -(CH₂)ₙ-N(Z)-(CH₂)ₘ-N(A)-(CH₂)ₚ-, in der n, m und p unabhängig voneinander für eine ganze Zahl von 1 bis 8, insbesondere von 1 bis 4, stehen können, n und m jedoch bevorzugt für die gleiche Zahl stehen, und Z und A unabhängig voneinander für Wasserstoff, eine C₁₋₈-, insbesondere C₁₋₄-,Alkylgruppe, eine C₁₋₈-, insbesondere C₁₋₄-, Mono-hydroxyalkylgruppe, eine C₂₋₈-, insbesondere
   C₂₋₄-, Dihydroxyalkylgruppe oder eine eine C₃₋₈-, insbesondere C₃₋₄-, Trihydroxyalkyl gruppe stehen,
- gegebenenfalls an den Alkylketten substituierte Ethergruppen, insbesondere solche der allgemeinen Formel -(CH₂)ₙ-O-(CH₂)ₘ-, in der n und m unabhängig voneinander für eine ganze Zahl von 1 bis 8, insbesondere von 1 bis 4, stehen können, jedoch bevorzugt für die gleiche Zahl stehen,
- gegebenenfalls an den Alkylketten substituierte Polyethergruppen, insbesondere solche der allgemeinen Formel -(CH₂)ₙ-O-(CH₂)ₘ-O-(CH₂)ₘ-O-(CH₂)ₙ-, in der n und m unabhängig voneinander für eine ganze Zahl von 1 bis 8, insbesondere von 1 bis 4, stehen können, jedoch bevorzugt für die gleiche Zahl stehen,
- schwefelhaltige Gruppen. insbesondere Gruppen der allgemeinen Formel -(CH₂)ₙ-S(O)ₒ-(CH₂)ₘ-, in der n und m unabhängig voneinander für eine ganze Zahl von 1 bis 8. insbesondere von 1 bis 4. stehen können, jedoch bevorzugt für die gleiche Zahl stehen, und o für 0, 1 oder 2 steht.

Die Spacer S sind in den erfindungsgemäßen Hybrid-Farbstoffen über ihre beiden freien Bindungen mit den Gruppen X und Y so verknüpft, daß sie jeweils als Substituent an die Stelle eines Wasserstoffatoms der Farbstoff- bzw. Farbstoffvorprodukt-Moleküle treten, die den Gruppen X und Y zugrunde liegen.

Gemäß einer ersten bevorzugten Ausführungsform tritt die Spacergruppe S als Substituent an die Stelle eines Wasserstoffatoms, das direkt an ein Ringsystem der Gruppe X oder Y gebunden ist. Beispiele für solche Ringsysteme sind
- aromatische und cycloaliphatische Kohlenwasserstoff-Ringsysteme, insbesondere Benzol-, Naphthalin-. Anthracen-, Naphthochinon- und Anthrachinon-Systeme
- heterocyclische Ringsysteme, insbesondere Pyridin-, Pyrazol-, Pyrimidin-, Indol- und Indolinsysteme.

Gemäß einer zweiten, bevorzugten Ausführungsform tritt die Spacergruppe S als Substituent an die Stelle eines Wasserstoffatoms einer primären oder sekundären Aminogruppe, die direkt oder über eine aliphatische Kohlenwasserstoffgruppe an ein aromatisches, cycloaliphatisches oder heterocyclisches Ringsystem gebunden ist.

Gemäß einer dritten Ausführungsform tritt die Spacergruppe S als Substituent an die Stelle des Wasserstoffatoms einer Hydroxygruppe, die direkt oder über eine aliphatische Kohlenwasserstoffgruppe an ein aromatisches, cycloaliphatisches oder heterocyclisches Ringsystem gebunden ist.

Die erfindungsgemäßen Hybridfarbstoffe eignen sich hervorragend zum Färben keratinischer Fasern. Unter keratinischen Fasern sind dabei Pelze, Wolle, Federn und insbesondere menschliche Haare zu verstehen. Einer Verwendung auch auf anderen Gebieten, insbesondere in der Farbphotographie. steht aber nichts entgegen.

Ein zweiter Gegenstand der vorliegenden Erfindung sind daher Mittel zum Färben keratinischer Fasern, insbesondere menschlicher Haare, die einen Hybridfarbstoff gemäß Struktur (I) enthalten. Selbstverständlich umfaßt die erfindungsgemäße Lehre auch solche Mittel, die Kombinationen von mehr als einem Hybridfarbstoff der Struktur (I) enthalten.

Die erfindungsgemäßen Mittel zum Färben menschlicher Haare können weiterhin alle für solche Mittel üblichen Bestandteile enthalten.

Bevorzugt enthalten die erfindungsgemäßen Färbemittel mindestens einen weiteren Farbstoff, ein weiteres Farbstoffvorprodukt und/oder ein Indol- oder Indolin-Derivat als Melanin-Vorstufe.

In einer ersten Ausführungsform sind solche Färbemittel besonders bevorzugt, die neben einem Hybridfarbstoff der Struktur (I) mindestens ein Oxidationsfarbstoffvorprodukt enthalten. Diese Oxidationsfarbstoffvorprodukte können sowohl vom Kupplertyp als auch vom Entwicklertyp sein.

Als Oxidationsfarbstoffvorprodukte vom Entwicklertyp kommen beispielsweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate in Betracht.

Erfindungsgemäß bevorzugte Entwicklerkomponenten sind p-Phenylendiamin, p-Toluylendiamin, p-Aminophenol, 2-Chlor-4-aminophenol, o-Aminophenol, 1-(2'-Hydroxyethyl)-2.5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylamino-4-amino-phenol, 4,4'-Diaminodiphenylamin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 931 bzw. WO 94/08970 wie z. B. 4,5-Diamino-1-(2'-hydroxyethyl)-pyrazol. Besonders bevorzugt sind p-Phenylendiamin, p-Toluylendiamin, p-Aminophenol, -1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin und 4-Hydroxy-2,5,6-triaminopyrimidin.

Als Oxidationsfarbstoffvorprodukte vom Kupplertyp kommen beispielsweise m-Phenylendiaminderivate, Naphthole, Resorcin und Resorcinderivate, Pyrazolone und m-Aminophenolderivate in Betracht.

Erfindungsgemäß bevorzugte Kupplerkomponenten sind
- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, 3-(Dimethylamino)-phenol, N-Cyclopentyl-3-aminophenol. 1,3-Dihydroxy-5-(methylamino)-benzol, 3-(Ethylamino)-4-methylphenol und 2,4-Dichlor-3-aminophenol,
- o-Aminophenol und dessen Derivate wie 4-Chlor-o-aminophenol,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-3,4-diaminopyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 3,4-Methylendioxyphenol, 1-Hydroxy-3,4-methylendioxybenzol, 1-Amino-3,4-methylendioxybenzol und 1-(2'-Hydroxyethyl)-amino-3,4-methylendioxybenzol,

Erfindungsgemäß besonders bevorzugte Kuppler sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-diaminopyridin, 2,4-Diaminophenoxyethanol, 1,3-Bis-(2,4-diaminophenoxy)-propan, 2-Aminomethyl-3-amino-6-methoxypyridin und 2,6-Dihydroxy-3,4-dimethylpyridin.

Im Rahmen dieser Ausführungsform kann bevorzugt sein:
- Hybridfarbstoff der Struktur (I) mit einer Gruppe Y, abgeleitet von einem Oxidationsfarbstoffvorprodukt vom Kupplertyp, in Kombination mit mindestens einem weiteren Oxidationsfarbstoffvorprodukt vom Entwicklertyp
- Hybridfarbstoff der Struktur (I) mit einer Gruppe Y. abgeleitet von einem Oxidationsfarbstoffvorprodukt vom Entwicklertyp, in Kombination mit mindestens einem weiteren Oxidationsfarbstoffvorprodukt vom Kupplertyp
- Hybridfarbstoff der Struktur (I) mit einer Gruppe Y, abgeleitet von einem Derivat des Indols oder Indolins als Vorläufer des Melanins, in Kombination mit mindestens einem weiteren Oxidationsfarbstoffvorprodukt vom Kupplertyp

In einer zweiten Ausführungsform sind solche Färbemittel bevorzugt, die neben einem Hybridfarbstoff der Struktur (I) mindestens einen direktziehenden Farbstoff enthalten.

Solche erfindungsgemäß einzusetzenden direktziehenden Farbstoffe sind beispielsweise Nitrophenylendiamine, Nitroaminophenole, Azofarbstoffe, Anthrachinone und Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure. 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die kationischen direktziehenden Farbstoffe, die unter dem Warenzeichnen Arianor® vertrieben werden, sind besonders bevorzugte direktziehende Farbstoffe.

Weiterhin können die erfindungsgemäßen Mittel auch in der Natur vorkommende Farbstoffe wie beispielsweise Henna rot, Henna neutral, Kamillenblüte, Sandelholz, schwarzer Tee, Faulbaumrinde, Salbei, Blauholz, Krappwurzel, Catechu, Sedre und Alkannawurzel enthalten.

Die erfindungsgemäßen Mittel gemäß dieser Ausfühnmgsform enthalten die direktziehenden Farbstoffe bevorzugt in einer Menge von 0,01 bis 20 Gew.-%, bezogen auf das gesamte Färbemittel.

In einer dritten Ausführungsform sind solche Färbemittel besonders bevorzugt, die neben einem Hybridfarbstoff der Struktur (I) mindestens ein Derivat des Indols oder Indolins als Vorläufer des Melanins enthalten.

Erfindungsgemäß bevorzugt sind solche Indole und Indoline, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Verbindungen mit zwei dieser Gruppen, insbesondere zwei Hydroxygruppen, von denen eine oder beide verethert oder verestert sind, sind besonders bevorzugt.

Erfindungsgemäß besonders bevorzugte Farbstoffvorprodukte sind Derivate des Indolins, wie das 5,6-Dihydroxyindolin, N-Methyl-5,6-dihydroxyindolin, N-Ethyl-5,6-dihydroxyindolin. N-Propyl-5,6-dihydroxyindolin, N-Butyl-5,6-dihydroxyindolin, 5,6-Dihydroxyindolin-2-carbonsäure, 5-Hydroxyindolin, 6-Hydroxyindolin, 5-Aminoindolin, 6-Aminoindolin und 4-Aminoindolin.

Ganz besonders bevorzugt sind Derivate des 5,6-Dihydroxyindolins der Formel (IIIa), 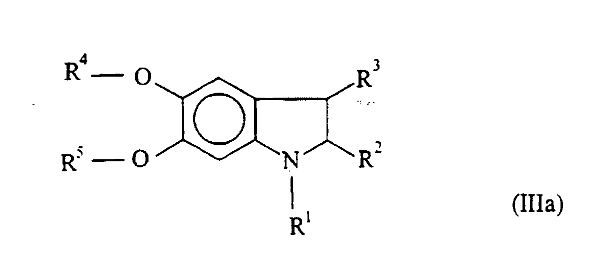 in der unabhängig voneinander
- R¹: steht für Wasserstoff, eine C1-C4-Alkylgruppe, eine C1-C4-Hydroxy-alkylgruppe oder eine C3-C6-Cycloalkylgruppe,
- R²: steht für Wasserstoff oder eine -COOH-Gruppe, wobei die -COOH-Gruppe auch als Salz mit einem physiologisch verträglichen Kation vorliegen kann,
- R³: steht für Wasserstoff oder eine C1-C4-Alkylgruppe,
- R⁴: steht für Wasserstoff, eine C1-C4-Alkylgruppe oder eine Gruppe -CO-R⁶, in der R⁶ steht für eine C1-C4-Alkylgruppe oder eine gegebenenfalls substituierte Phenylgruppe, und
- R⁵: steht für eine der unter R⁴ genannten Gruppen,
oder ein physiologisch verträgliches Salz dieser Verbindungen mit einer organischen oder anorganischen Säure.

Erfindungsgemäß bevorzugte Vertreter sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin. Der Grundkörper, das 5,6-Dihydroxyindolin, ist ganz besonders bevorzugt.

Erfindungsgemäß bevorzugte Indole sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 5-Hydroxyindol, 6-Hydroxyindol, 5-Aminoindol, 6-Aminoindol und 4-Aminoindol.

Besonders bevorzugt sind Derivate des 5,6-Dihydroxyindols der Formel (IIIb), 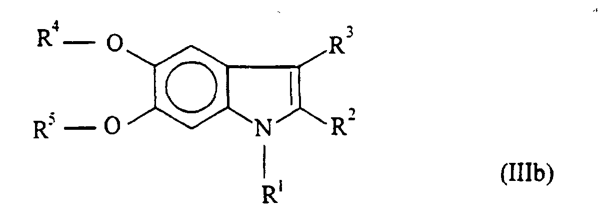 in der unabhängig voneinander
- R¹: steht für Wasserstoff, eine C1-C4-Alkylgruppe, eine C1-C4-Hydroxyalkylgruppe oder eine C3-C6-Cycloalkylgruppe,
- R²: steht für Wasserstoff oder eine -COOH-Gruppe, wobei die -COOH-Gruppe auch als Salz mit einem physiologisch verträglichen Kation vorliegen kann,
- R³: steht für Wasserstoff, eine C1-C4-Alkylgruppe oder eine Gruppe -CH₂-NR⁷R⁸, in der R⁷ und R⁸ unabhängig voneinander stehen für Wasserstoff oder eine C1-C4-Alkylgruppe,
- R⁴: steht für Wasserstoff, eine C1-C4-Alkylgruppe oder eine Gruppe -CO-R⁶, in der R⁶ steht für eine C1-C4-Alkylgruppe oder eine gegebenenfalls substituierte Phenylgruppe, und
- R⁵: steht für eine der unter R⁴ genannten Gruppen,
oder ein physiologisch verträgliches Salz dieser Verbindungen mit einer organischen oder anorganischen Säure.

Erfindungsgemäß bevorzugte Vertreter sind das 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol. Der Grundkörper, das 5,6-Dihydroxyindol, ist ganz besonders bevorzugt.

Selbstverständlich umfaßt die vorliegende Erfindung auch Mittel, die mehr als ein Indolin- oder Indol-Derivat oder Mischungen von Indolin- und Indol-Derivaten enthalten.

Die Indol- oder Indolin-Derivate sind in den erfindungsgemäßen Mitteln üblicherweise in Mengen von 0,05-10 Gew.-%, vorzugsweise 0,2-5 Gew.-% enthalten.

Im Rahmen dieser Ausführungsform kann bevorzugt sein:
- Hybridfarbstoff der Struktur (I) mit einer Gruppe Y, abgeleitet von einem Oxidationsfarbstoffvorprodukt vom Kupplertyp, in Kombination mit mindestens einem Derivat des Indols oder Indolins als Vorläufer des Melanins,
- Hybridfarbstoff der Struktur (I) mit einer Gruppe Y. abgeleitet von einem Oxidationsfarbstoffvorprodukt vom Entwicklertyp, in Kombination mit mindestens einem Derivat des Indols oder Indolins als Vorläufer des Melanins.

Im Rahmen der genannten Ausführungsformen ist es nicht erforderlich, daß die Oxidationsfarbstoffvorprodukte, direktziehenden Farbstoffe oder Melanin-Vorläufer jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die in den erfindungsgemäßen Mitteln enthaltenen Hybridfarbstoffe, Kuppler- und Entwicklerkomponenten mit Aminogruppen, sowie Indolin- und Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bezüglich der in den erfindungsgemäßen Haarfärbe- und Tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8. Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986. sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V.. Mannheim, Bezug genommen.

Erfindungsgemäße Mittel, enthaltend einen Hybridfarbstoff der Struktur (I), in der sich die Gruppe Y von einem Melaninvorläufer von Indol- oder Indolin-Typ ableitet, enthalten gemäß einer bevorzugten Variante dieser Ausführungsform außer den genannten Hvbridfarbstoffen keine weiteren Farbstoffe oder Farbstoffvorprodukte.

Gemäß einer weiteren bevorzugten Variante enthalten insbesondere erfindungsgemäße Mittel mit einem Hybridfarbstoff der Struktur (I), in der sich die Gruppe Y von einem Melaninvorläufer von Indol- oder Indolin-Typ ableitet, und/oder mit einem Melanin-Vorläufer weiterhin mindestens eine Aminosäure oder ein Oligopeptid.

Aminosäuren im Sinne der Erfindung sind solche Substanzen, die mindestens eine Aminogruppe und mindestens eine -COOH- oder -SO₃H-Gruppe aufweisen.

Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Omithin und Histidin besonders bevorzugt.

Die Aminosäuren können den erfindungsgemäßen Mitteln bevorzugt in freier Form zugegeben werden. Es ist auch möglich, die Aminosäuren in Salzform einzusetzen. Bevorzugte Salze sind dann die Verbindungen mit Halogenwasserstoffsäuren, insbesondere die Hydrochloride und die Hydrobromide.

Ganz besonders bevorzugte Aminosäuren sind Lysin und insbesondere Arginin, insbesondere in freier Form, aber auch als Hydrochlorid eingesetzt.

Weiterhin können die Aminosäuren auch in Form von Oligopeptiden und Proteinhydrolysaten eingesetzt werden, wenn sichergestellt ist, daß die erforderlichen Mengen an Verbindungen, die der erfindungsgemäßen Definition der Aminosäuren entsprechen. darin enthalten sind. In diesem Zusammenhang wird auf die Offenbarung der DE-OS 22 15 303 verwiesen, auf die ausdrücklich Bezug genommen wird.

Selbstverständlich umfaßt die Erfindung auch solche Mittel, die zwei und mehr Aminosäuren oder Oligopeptide enthalten. Bevorzugt sind dabei Kombinationen von Arginin mit einer weiteren Aminosäure oder einem Oligopeptid.

Die erfindungsgemäßen Mittel enthalten die Aminosäure bzw. das Oligopeptid bevorzugt in Mengen von 0,1 bis 10 Gew.-%, insbesondere 1 bis 4 Gew.-%, bezogen auf das gesamte Mittel.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkalioder Erdalkalihydroxide, Ammoniak oder organische Amine.

Gemäß einer speziellen Ausführungsform der vorliegenden Erfindung dient die Aminosäure oder das Oligopeptid nicht nur der Intensivierung der Ausfärbung, sondern übernimmt auch, zumindest teilweise, die Funktion des Alkalisierungsmittels. Im Rahmen dieser Ausführungsform werden daher bevorzugt solche Aminosäuren und Oligopeptide eingesetzt. deren 2,5 Gew.-%ige Lösungen in Wasser einen pH-Wert von 9 und größer aufweisen. Solche Aminosäuren sind die bevorzugt eingesetzten Verbindungen Arginin und Lysin. Im Rahmen dieser Ausführungsform wird das weitere Alkalisierungsmittel bevorzugt ausgewählt aus der Gruppe, die von Monoethanolamin, Monoisopropanolamin. 2-Amino-2-methyl-propanol, 2-Amino-2-methyl-1,3-propandiol, 2-Amino-2-ethyl-1,3-propandiol. 2-Amino-2-methylbutanol und Triethanolamin sowie Alkali- und Erdalkalimetallhydroxiden gebildet wird. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist im Rahmen dieser Ausführungsform bevorzugt.

Besonders vorteilhafte Eigenschaften wurden bei Mitteln gefunden, bei denen die Aminosäure oder das Oligopeptid und das weitere Alkalisierungsmittel in einem Verhältnis von 1:5 bis 5:1 vorlagen. Mengenverhältnisse von 1:2 bis 2:1 haben sich als besonders geeignet erwiesen.

Zur Herstellung der erfindungsgemäßen Färbemittel werden die oben genannten zwingenden und fakultativen Bestandteile in einen geeigneten, bevorzugt wasserhaltigen, Träger eingearbeitet. Solche Träger sind z. B. Cremes, Emulsionen, Gele oder auch tensidhaltige schäumende Lösungen, z. B. Shampoos, Schaumaerosole oder andere Zubereitungen, die für die Anwendung auf dem Haar geeignet sind. Diese Formulierungsformen werden mit den bereits oben erwähnten Alkalisierungsmitteln oder geeigneten Säuren, wie insbesondere Genußsäuren wie Citronensäure, Weinsäure, Milchsäure und Essigsäure, bevorzugt auf einen pH-Wert von 5 bis 11, insbesondere von 7 bis 10, eingestellt.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe.
- Acyltauride mit 10 bis 18 C-Atomen in der Acylgruppe.
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylen glykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungs produkte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat. N-Acyl-aminopropyl-N,N-dimethylammoniumglycinate. beispielsweise das Kokosacylamino-propyl-dimethyl-ammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacyl-aminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C8-C18-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren. N-Hydroxyethyl-N-alkylamidopropylglycine, N-Alkyltaurine. N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C 12-18-Acylsarcosin.

Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C12-C22-Fettsäuremono- und -diester von Anlagerungsprodukten von 1 bis 30 Mol Ethylenoxid an Glycerin.
- C8-C22-Alkylmono- und -oligoglycoside, deren ethoxylierte Analoga und deren Ester z. B. mit Weinsäure und Citronensäure,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldi-methylammoniumchloride und Trialkylmethylammoniumchloride, z. B. Cetyltrimethyl-ammoniumchlorid, Stearyltrimethylammoniumchlorid, Distearyldimethylammonium-chlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methyl-hydroxyalkyl-dialkoyloxyalkyl-ammonium-methosulfate sowie die entsprechenden Produkte. die unter dem Warenzeichen Dehyquart® im Handel erhältlich sind.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Erfindungsgemäß kann die Verwendung anionischer Tenside in Kombination mit zwitterionischen Tensiden besonders bevorzugt sein.

Ebenfalls erfindungsgemäß bevorzugt sind solche Mittel, die zusätzlich ein kationisches Polymer enthalten.

Unter den kationischen Polymeren sind dabei die permanent kationischen Polymere bevorzugt. Als "permanent kationisch" werden erfindungsgemäß solche Polymeren bezeichnet, die unabhängig vom pH-Wert des Mittels eine kationische Gruppe aufweisen. Dies sind in der Regel Polymere. die ein quartäres Stickstoffatom, beispielsweise in Form einer Ammoniumgruppe. enthalten.
Bevorzugte kationische Polymere sind beispielsweise
- quatemisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- Polysiloxane mit quaternären Gruppen, wie beispielsweise die im Handel erhältlichen Produkte Q2-7224 (Hersteller: Dow Coming; ein stabilisiertes Trimethylsilylamodimethicon), Dow Corning® 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80),
- Kationische Guar-Derivate, wie insbesondere die unter den Handelsnamen Cosmedia®Guar und Jaguar® vertriebenen Produkte,
- Polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,
- sowie die unter den Bezeichnungen
- Polyquatemium 2,
- Polyquatemium 17,
- Polyquatemium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), Polyquaternium-32, Polyquaternium-35 und Polyquaternium-37 (Handelsprodukte z. B. Salcare® SC 92 und Salcare®SC 95) bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Erfindungsgemäß bevorzugte kationische Polymere sind quatemisierte Cellulose-Derivate, polymere Dimethyldiallylammoniumsalze, Polyquaternium-27 und deren Copolymere sowie Polymere vom Typ Polyquaterniuim-2. Kationische Cellulose-Derivate, insbesondere das Handelsprodukt Polymer®JR 400, und Polymere vom Typ Polyquaternium-2, insbesondere das Handelsprodukt Mirapol®A-15, sind ganz besonders bevorzugte kationische Polymere.

Die kationischen Polymere sind in den erfindungsgemäßen Mitteln bevorzugt in Mengen von 0,05 bis 5 Gew.-%, bezogen auf die gesamte Zubereitung, enthalten.

In vielen Fällen können als Alternative zu den kationischen Polymeren auch Ampho-Polymere eingesetzt werden. Unter dem Oberbegriff Ampho-Polymere sind amphotere Polymere, d. h. Polymere, die im Molekül sowohl freie Aminogruppen als auch freie -COOH- oder SO₃H-Gruppe enthalten und zur Ausbildung innerer Salze befähigt sind, zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Grupp und quartäre Ammoniumgruppen enthalten. Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure. Methacrylsäure und deren einfachen Estern darstellt. Ebenfalls bevorzugte Amphopolymere setzen sich aus ungesättigten Carbonsäure (z. B. Acryl- und Methacrylsäure), kationisch derivatisierten ungesättigten Carbonsäure (z. B. Acrylamidopropyl-trimethyl-ammoniumchlorid) und gegebenenfalls weiteren ionischen oder nichtionogenen Monomeren zusammen, wie beispielsweise in der deutschen Offenlegungsschrift 39 29 973 und dem dort zitierten Stand der Technik zu entnehmen sind. Ebenfalls erfindungsgemäß einsetzbar sind Terpolymere von Acrylsäure, Methylacrylat und Methacrylamidopropyltrimoniumchlorid, wie sie unter der Bezeichnung Merquat®2001 N im Handel erhältlich sind, sowie das Handelsprodukt Merquat®280.

Ebenfalls bevorzugt enthalten die erfindungsgemäßen Mittel mindestens ein nichtionogenes oder anionisches Polymer mit verdickenden Eigenschaften. Bevorzugt sind dabei, gegebenenfalls vernetzte. Polyacrylsäuren, Cellulose-Derivate, z. B. Methylcellulose, Hydroxyalkylcellulose und Carboxymethylcellulose, und Xanthan-Gum.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Gummi arabicum, Karaya-Gummi, Johannisbrotkernmehl, Leinsamengummen, Dextrane, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline. sowie Silikonöle.
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin, Diethylenglykol, Ethoxybutanol und Butoxyethanol sowie Benzylalkohol,
- Antischuppenwirkstoffe wie Piroctone Olamine und Zink Omadine,
- weitere Substanzen zur Einstellung des pH-Wertes, wie beispielsweise α- und β-Hydroxycarbonsäuren,
- Wirkstoffe wie Panthenol, Pantothensäure. Allantoin, Pyrrolidoncarbonsäuren und deren Salze, Pflanzenextrakte und Vitamine.
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft,
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Bezüglich der weiteren Bestandteile der erfindungsgemäßen Färbemittel wird auch ausdrücklich auf die dem Fachmann bekannten Monographien, z. B. Umbach, Kosmetik, 2. Auflage, Georg Thieme Verlag, Stuttgart New York, 1995, und Kh. Schrader, Grundlagen und Rezepturen der Kosmetika, 2. Auflage, Hüthig Buch Verlag, Heidelberg, 1989, verwiesen.

Zur Ausbildung der Färbung auf der keratinischen Faser können alle bekannten Verfahren angewendet werden.

Haarfärbemittel mit einem oder mehreren Hybridfarbstoffen, bei denen die Gruppen X und Y jeweils von direktziehenden Farbstoffen abgeleitet sind und die keine weiteren Farbstoffvorprodukte enthalten, können wie übliche Tönungsmittel auf das Haar aufgetragen werden. Üblicherweise werden diese Mittel nach einer gewissen Einwirkzeit wieder abgewaschen und das Haar gewünschtenfalls noch einer Nachbehandlung unterzogen. Bei Verwendung in einer Haar-Mascara kann das Färbemittel aber auch mittels einer Auftragehilfe auf das Haar, insbesondere aber auch auf einzelne Haarsträhnen, aufgetragen und auf diesem belassen werden.

Gemäß einer weiteren Ausführungsform erfolgt die Ausbildung der Färbung mit Luftsauerstoff als einzigem Oxidationsmittel. Diese Ausführungsform ist insbesondere dann bevorzugt, wenn die Gruppe Y des Hybridfarbstoffes sich von einem Vorläufer des Melanins oder luftoxidablen Entwickler- und Kupplerkomponenten ableitet, oder das Mittel Vorläufer des Melanins und/oder luftoxidable Farbstoffvorprodukte vom Kuppler- oder Entwicklertyp enthält. Als luftoxidable Verbindungen sind erfindungsgemäß solche Verbindungen oder Farbstoffvorprodukte zu verstehen, bei denen die oxidative Ausbildung der endgültigen Färbung allein mit Hilfe von Luftsauerstoff ohne Verwendung üblicher chemischer Oxidationsmittel erfolgen kann. Triaminobenzol-Derivate sind Beispiele für solche luftoxidablen Verbindungen.

Insbesondere in Fällen, in denen die Gruppe Y des Hybridfarbstoffes sich von einem Vorläufer des Melanins oder Entwickler- und Kupplerkomponenten ableitet, oder das Mittel Vorläufer des Melanin und/oder Farbstoffvorprodukte vom Kuppler- oder Entwicklertyp enthäl, kann in vielen Fällen die Mitverwendung eines chemischen Oxidationsmittels bevorzugt sein. Dies gilt auch, wenn neben der Färbung ein Authelleffekt an menschlichem Haar gewünscht ist. Als Oxidationsmittel kommen dann insbesondere Wasserstoffperoxid oder dessen Anlagerungsprodukte an Harnstoff, Melamin oder Natriumborat in Frage.

Zweckmäßigerweise wird die Zubereitung des Oxidationsmittels unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 5 bis 11 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein. Gemäß einer besonderen Ausführungsform dieses Verfahrens erfolgt die Ausbildung der endgültigen Färbung durch mehrmaliges Aufbringen des Mittels und jeweils anschließender Luftoxidation. Das jeweilige Aufbringen des Mittels erfolgt dabei bevorzugt in Abständen, die zwischen etwa einem Tag und etwa zwei Wochen liegen. Dadurch können sehr gezielt spezielle Nuancen erhalten werden.

Unabhängig davon, welches der oben genannten Verfahren zur Anwendung des erfindungsgemäßen Mittels gewählt wird, kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Weiterhin ist es erfindungsgemäß möglich, die Oxidation mit Hilfe von Enzymen durchzuführen. Dabei können die Enzyme (Enzymklasse 1: Oxidoreduktasen) Elektronen aus geeigneten Entwicklerkomponenten (Reduktionsmittel) auf Luftsauerstoff übertragen. Bevorzugt sind Oxidasen wie Tyrosinase und Laccase aber auch Glucoseoxidase, Ascorbat-Oxidase Uricase oder Pyruvatoxidase. Weiterhin können die Enzyme zur Verstärkung der Wirkung geringer Mengen vorhandener Oxidationsmittel dienen. Ein Beispiel für ein derartiges enzymatisches Verfahren stellt das Vorgehen dar, die Wirkung geringer Mengen (z. B. 1% und weniger, bezogen auf das gesamte Mittel) Wasserstoffperoxid durch Peroxidasen zu verstärken.

Ein weiterer Gegenstand der Erfindung ist die Verwendung eines Hybridfarbstoffes der Struktur (I) oder einer Mischung dieser Hybridfarbstoffe zum Färben keratinischer Fasern, insbesondere menschlicher Haare.

Weiterhin hat sich gezeigt, daß die erfindungsgemäßen Hybridfarbstoffe sich auch hervorragend zur Anfärbung, insbesondere zur Bräunung, der menschlichen Haut eignen. Ein weiterer Gegenstand der Erfindung ist daher die Verwendung eines Hybridfarbstoffes der Struktur (I) oder einer Mischung -dieser Hybridfarbstoffe zum Färben der menschlichen Haut.

Die nachfolgenden Beispiele sollen den Erfindungsgegenstand näher erläutern.

### Beispiele

### 1. Synthesen

### 1.1. 1-((3-((4-Aminophenyl)amino)-propyl)amino)anthracen-9,10-dion (Hybridfarbstoff A)

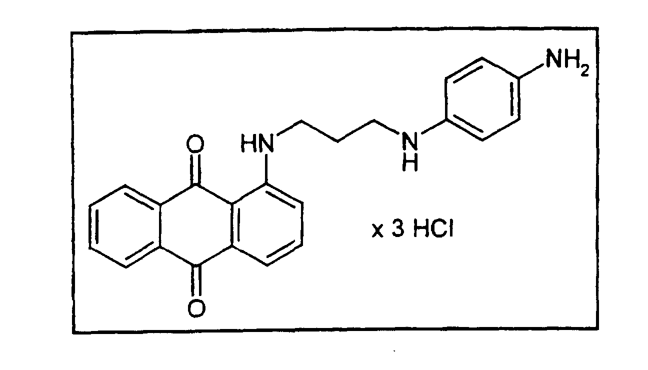

- X: abgeleitet von einem direktziehenden Farbstoff (1-Aminoanthracen-9,10-dion)
- Y: abgeleitet von einem Oxidationsfarbstoffvorprodukt vom Entwicklertyp (p- Phenylendiamin)
- S: Alkylengruppe (-CH₂-CH₂-CH₂-)

### 1. Stufe

Ein Gemisch aus 3,73 g (0,015 mol) 1-Chloranthrachinon, 3,00 g (0,015 mol) N-(p-Nitrophenyl)propylendiamin, 1,54 g (0,011 mol) Kaliumcarbonat und 0,13 g Cu-Pulver in 40 ml DMSO wurde unter Rühren und Stickstoffatmosphäre 1,5 h bei 120 - 130 °C zur Reaktion gebracht. Nach Erkalten wurde der Rückstand abfiltriert und aus Aceton/Wasser umkristallisiert. Man erhielt 1,4 g 1-((3-((4-Aminophenyl)amino)propyl)-amino)anthracen-9,10-dion (Schmelzpunkt: 183-185 °C) als rotes Pulver.

### 2. Stufe

Eine Lösung von 1,00 g (2,4 mmol) 1-((3-((4-Aminophenyl)amino)propyl)-amino)anthracen-9,10-dion in 340 ml Ethanol wurde in Gegenwart von 0,2 g Pd (5 %) auf Kohle bei 25 °C hydriert (1013 mbar). Nach beendeter H₂-Aufnahme wurde die Lösung mit verdünnter Salzsäuze angesäuert, vom Katalysator abfiltriert und zur Trockene eingeengt. Man erhielt einen roten Feststoff mit einem Schmelzpunkt oberhalb 170 °C (Zers.)

### 1.2. 1-Amino-4-((3-((4-amino-phenyl)amino)-propyl)amino)-anthracen-9,10-dion-2-sulfonsäure (Hybridfarbstoff B)

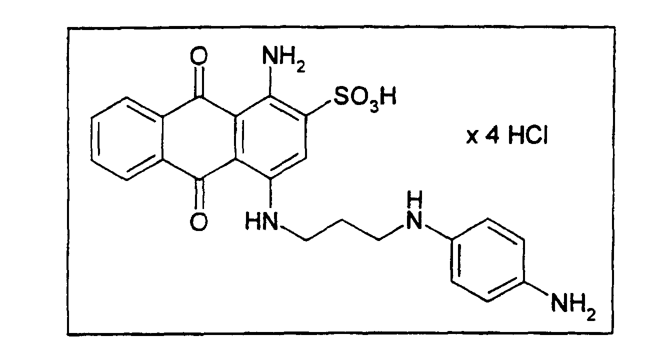

- X: abgeleitet von einem direktziehenden Farbstoff (1,4-Diaminoanthracen-9, 10-dion-2-sulfonsäure)
- Y: abgeleitet von einem Oxidationsfarbstoffvorprodukt vom Entwicklertyp (p-Phenylendiamin)
- S: Alkylengruppe (-CH₂-CH₂-CH₂-)

### 1. Stufe

Ein Gemisch aus 100,0 g (0,25 mol) Na-Salz der 1-Amino-4-brom-anthrachinon-2-sulfonsäure. 48,3 g (0,25 mol) N-(p-Nitrophenyl)propylendiamin, 51,8 g (0,37 mol) Kaliumcarbonat, und 0,7 g Cu-Pulver in 600 ml Wasser wurden unter Rühren und Stickstoffatmosphäre 3 h in der Siedehitze zur Reaktion gebracht. Nach Erkalten wurde der Rückstand abfiltriert und zunächst aus Ethanol/Wasser umkristallisiert. Der so erhaltene Feststoff wurde in 1.5 l Wasser gelöst und mit verdünnter Salzsäure auf pH 3,6 eingestellt. Hierbei fiel das Zwischenprodukt 1-Amino-4-((3-((4-nitro-phenyl)amino)-propyl)amino)-anthracen-9,10-dion-2-sulfonsäure in Form der freien Sulfonsäure als blauer Feststoff (Schmelzpunkt: 215 °C) aus.

### 2. Stufe

Eine Lösung von 15,0 g (0,03 mol) 1-Amino-4-((3-((4-nitro-phenyl)amino)-propyl)amino)-anthracen-9,10-dion-2-sulfonsäure in 900 ml Ethanol wurde in Gegenwart von 1,0 g Pd (5 %) auf Kohle bei 25 °C hydriert (1013 mbar). Nach beendeter H₂-Aufnahme wurde die Lösung mit verdünnter Salzsäure angesäuert, vom Katalysator abfiltriert und zur Trockene eingeengt. Man erhielt das Produkt in Form eines blauschwarzen Feststoffs mit einem Schmelzpunkt oberhalb 250 °C (Zers.).

### 1.3. 4-((2-((4-Amino-2-nitrophenyl)amino)ethyl)amino)phenol (Hybridfarbstoff C)

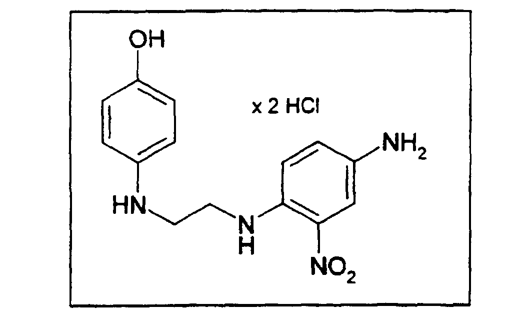

- X: abgeleitet von einem direktziehenden Farbstoff (4-Amino-2-nitroänilin)
- Y: abgeleitet von einem Oxidationsfarbstoffvorprodukt vom Entwicklertyp (p-Aminophenol)
- S: Alkylengruppe (-CH₂-CH₂-)

10.0 g (0,04 mol) p-(β-Aminoethylamino)-phenol-sulfat (hergestellt gemäß US 2.618,657), 8.42 g (42,5 mmol) 4-Fluor-3-nitroacetanilid und 6,05 g Natriumhydrogencarbonat wurden in 40 ml DMSO unter Rühren und Stickstoffatmosphäre 3,5 h bei einer Temperatur von 65 °C umgesetzt. Das Reaktionsgemisch wurde anschließend auf Eis gegeben und der Niederschlag isoliert. Dieser wurde in 80 ml verdünnter Salzsäure für 1 h refluxiert. Die Lösung wurde auf Eis gegeben und der Niederschlag 4-((2-((4-Amino-2-nitrophenyl)amino)ethyl)amino)phenol als grünbrauner Feststoff (Schmelzpunkt: 256 °C) isoliert.

### 1.4 4-((2-((2,4-Dinitrophenyl)amino)ethylamino)phenol-sulfat (Hybridfarbstoff D)

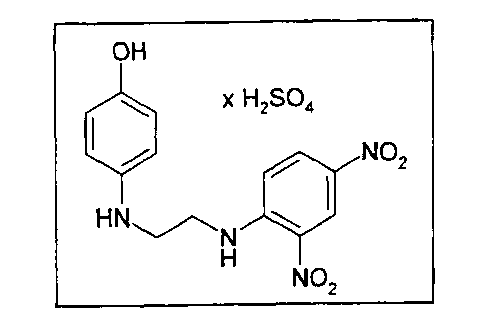

- X: abgeleitet von einem direktziehenden Farbstoff (2,4-Dinitroanilin)
- Y: abgeleitet von einem Oxidationsfarbstoffvorprodukt vom Entwickiertyp (p-Amino-phenol)
- S: Alkylengruppe (-CH₂-CH₂-)

### 1. Stufe

12,0 g (0,048 mol) p-(β-Aminoethylamino)-phenol-sulfat (US 2,618,657), 8,9 g (47,8 mmol) 2,4-Dinitrofluorbenzol und 6,05 g Kaliumcarbonat (0,057 mol) wurden in 150 ml Ethanol unter Rühren und Stickstoffatmosphäre 2,5 h in der Siedehitze umgesetzt. Anschließend wurde die Lösung abgekühlt und von den Salzen abfiltriert. Das Filtrat wurde mit verdünnter Schwefelsäure auf pH 2 eingestellt, der Niederschlag isoliert und aus Ethanol/Wasser umkristallisiert. Man erhielt das Produkt in Form brauner Kristalle mit einem Schmelzpunkt von 238 °C (Zers.).

### 1.5. (2-((2-Amino-4-nitrophenyl)amino)-ethyl)-naphthylamin (Habridfarbstoff E)

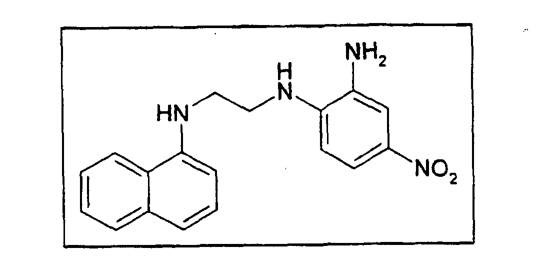

- X: abgeleitet von einem direktziehenden Farbstoff (2-Amino-4-nitroanilin)
- Y: abgeleitet von einem Oxidationsfarbstoffvorprodukt vom Kupplertyp (1-Naphthylamin)
- S: Alkylengruppe (-CH₂-CH₂-)

### 1. Stufe

10,0 g (38,6 mmol) N-(1-Naphthyl)-ethylendiamin-dihydrochlorid wurden in wenig Wasser gelöst und mit verdünnter Natronlauge neutral gestellt. Nach Zugabe von 120 ml Ethanol und 6,4 g Kaliumcarbonat (46,3 mmol) wurden 7,2 g (38,7 mmol) 2,4-Dinitrofluorbenzol tropfenweise zugegeben. Anschließend wurde 1 h refluxiert auf Raumtemperatur abgekühlt und der Niederschlag abgesaugt und mit Wasser/Ethanol gewaschen. Das Zwischenprodukt (2-(2,4-Dinitrophenyl)-amino)-ethyl)-naphthylamin wurde in Form roter Kristalle mit einem Schmelzpunkt von 263 °C erhalten.

### 2. Stufe

Zu einer Lösung von 3,7 g (10,5 mmol) (2-(2,4-Dinitrophenyl)-amino)-ethyl)-naphthylamin und 0,8 g NaOH in 100 ml Ethanol/67 ml Wasser wurde eine warme Lösung aus 2,5 g (10,5 mmol) Na₂S x 9H₂O und 0,67 g Schwefel in 10 ml Ethanol zugegeben. Nach 3 h refluxieren wurde heiß filtriert. Der aus dem Filtrat beim Abkühlen ausfallende Niederschlag wurde isoliert und mit Wasser/Ethanol gewaschen. Das Produkt wurde in Form roter Kristalle mit einem Schmelzpunkt von 149 °C erhalten.

### 1.6. (4-Amino-2-nitrophenyl)-((3,5-dimethoxyphenyl)methyl)amin (Hybridfarbstoff F)

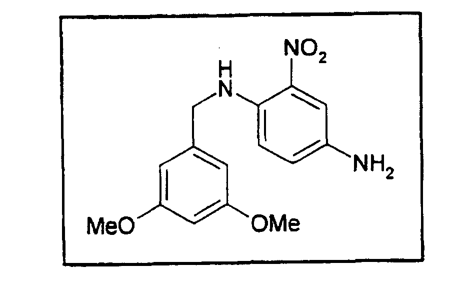

- X: abgeleitet von einem direktziehenden Farbstoff (4-Amino-2-nitroanilin)
- Y: abgeleitet von einem Oxidationsfarbstoffvorprodukt vom Kupplertyp (3,5-Dimethoxy-benzol)
- S: Alkylengruppe (-CH₂-)

### 1. Stufe

Eine Mischung aus 8,0 g (47,8 mmol) 3,5-Dimethoxybenzylamin, 7,47 g 4-Fluor-3-Nitroanilin (47,8 mmol) und 7,93 g Kaliumcarbonat (57,4 mmol) wurde unter Rühren und Stickstoffatmosphäre 1 h refluxiert. Beim Abkühlen fiel ein Niederschlag aus, der aus DMF umkristallisiert wurde. Das Produkt wurde in Form dunkelvioletter Kristalle mit einem Schmelzpunkt von 128 °C erhalten.

### 1.7. 2-(5,6-Dimethoxyindoliyl)-5-nitrophenylamin / 4-(5,6-Dimethoxyindoliyl)-3-nitrophenylamin (Hybridfarbstoff G)

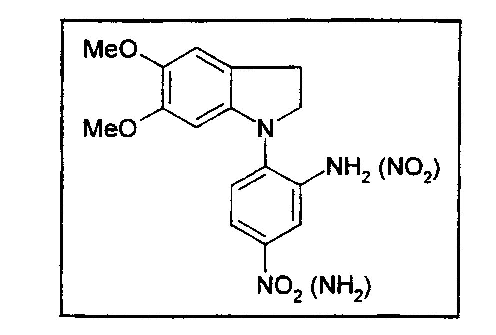

- X: abgeleitet von einem direktziehenden Farbstoff (3-Nitroanilin)
- Y: abgeleitet von einem Melanin-Vorläufer (5,6-Dimethoxyindolin)
- S: direkte Bindung

### 1. Stufe

Zu einer Mischung von 10.0 g 5,6-Dimethoxyindolin (55,8 mmol) und 11.57 g Kaliumcarbonat (83.7 mmol) wurden unter Rühren und Stickstoffatmosphäre bei Raumtemperatur 10,38 g (55,8 mmol) 1-Fluor-2,4-Nitrobenzol gegeben. Nach 3 h Rühren wurde der Niederschlag isoliert und mit Wasser gewaschen. Das Zwischenprodukt 1-(2,4-Dinitrophenyl)-5,6-dimethoxyindolin wurde als brauner Feststoff mit einem Schmelzpunkt von 219 °C erhalten.

### 2. Stufe

Zu einer Lösung von 7,2 g (20,9 mmol) 1-(2,4-Dinitrophenyl)-5,6-dimethoxyindolin und 0,8 g NaOH in 200 ml Ethanol/134 ml Wasser wurde eine warme Lösung aus 5,0 g (21 mmol) Na₂S x 9H₂O und 1,34 g Schwefel in 10 ml Ethanol zugegeben. Nach 3 h refluxieren wurde heiß filtriert. Das Filtrat wurde zur Hälfte eingeengt und mit konzentrierter Salzsäure auf pH 7 eingestellt. Der beim Abkühlen ausfallende Niederschlag wurde isoliert und mit Wasser/Ethanol gewaschen. Das Produkt wurde als Isomerengemisch (Reduktion bevorzugt an ortho gegenüber para-Position im Verhältnis ca. 4:1, mittels NMR-Spektroskopie bestimmt) in Form brauner Kristalle mit einem Schmelzbereich von ca. 115-130 °C erhalten.

### 2. Ausfärbungen

Es wurde zunächst eine Cremebasis folgender Zusammensetzung hergestellt [alle Angaben sind, soweit nicht anders vermerkt, in g]:

| | |
|---|---|
| Talgfettalkohol | 17,0 |
| Lorol^{R}techn ¹ | 4,0 |
| Texapon^{R}N 28² | 40,0 |
| Dehyton^{R}K³ | 25,0 |
| Eumulgin^{R}B 2⁴ | 1,5 |
| destilliertes Wasser | 12,5 |

| | |
|---|---|
| ¹ C₁₂₋₁₈-Fettalkohol (HENKEL) | |
| ² Natriumlaurylethersulfat (ca. 28 % Aktivsubstanz; CTFA-Bezeichnung: Sodium Laureth Sulfate) (HENKEL) | |
| ³ Fettsäureamid-Derivat mit Betainstruktur der Formel R-CONH(CH₂)₃N⁺(CH₃)₂CH₂COO⁻ (ca. 30 % Aktivsubstanz; CTFA-Bezeichnung Cocoamidopropyl Betaine) (HENKEL) | |
| ⁴ Cetylstearylalkohol mit ca. 20 Mol EO (CTFA-Bezeichnung: Ceteareth-20) (HENKEL) | |

Auf Basis dieser Creme wurde dann folgende Haarfärbecremeemulsion hergestellt:

| | |
|---|---|
| Cremebasis | 50,0 |
| Entwicklerkomponente | 7,5 mmol |
| Kupplerkomponente | 7,5 mmol |
| Na₂SO₃ (Inhibitor) | 1,0 |
| (NH₄)₂SO₄ | 1,0 |
| konz. Ammoniaklösung | ad pH 10 |
| Wasser | ad 100 |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt.

Die oxidative Entwicklung der Färbung wurde mit 3 %iger Wasserstoffperoxidlösung als Oxidationslösung durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrauten aber nicht besonders vorbehandelten Menschenhaar aufgetragen und dort 30 Minuten bei 32 °C belassen. Nach Beendigung des Färbeprozesses wurde das Haar gespült, mit einem üblichen Haarwaschmittel gewaschen und anschließend getrocknet.

Als weitere Farbstoffe/Farbstoffvorprodukte wurden folgende Verbindungen eingesetzt:

Oxidationsfarbstoffvorprodukte vom Entwicklertyp:
- E1: p-Toluylendiamin
- E2: 4-Aminophenol
- E3: 2,4,5,6-Tetraaminopyrimidin

Oxidationsfarbstoffvorprodukte vom Kupplertyp
- K1: 2,4-Diaminophenoxyethanol
- K2: 1-Naphthol
- K3: 1,3-Bis-(2,4-diaminophenoxy)-propan
- K4: 5-Amino-2-methylphenol
- K5: 4-Chlorresorcin
- K6: Resorcin
- K7: 2-Methyl-5-(2'hydroxyethyl)-aminophenol
- K8: 3,4-Methylendioxyphenol
- K9: 2-Chlor-6-methyl-3-aminophenol)

Die Ergebnisse der Ausfärbungen sind in der folgenden Tabelle zusammengestellt:

| **Hybridfarbstoff** | **weiteres Farbstoffvorprodukt** | **Nuance des gefärbten Haares** |
|---|---|---|
| A | K1 | blaugrau |
| A | K2 | dunkelblau |
| B | K1 | schwarzblau |
| B | K3 | nordischblau |
| B | K2 | dunkelblau |
| C | K2 | rubinrot |
| C | K3 | dunkelpurpur |
| C | K6 | mattrot |
| C | K4 | mattrot |
| C | K5 | mattrot |
| C | K7 | graurubin |
| C | K8 | dunkelrubin |
| C | K9 | mattrot |
| D | K6 | hellgelb |
| D | K4 | gelborange |
| D | K3 | gemsgelb |
| D | K5 | pastellgelb |
| E | E1 | lehmfarbig |
| F | E1 | mattrot |
| G | E1 | braunorange |
| G | E3 | cremefarben |
| G | E2 | goldblond |
| G | - | elfenbeinfarben^{a} |

| | | |
|---|---|---|
| ^{a} In diesem Fall erfolgte die Ausfärbung ohne Wasserstoffperoxid | | |

## Patentansprüche

1. Hybridfarbstoff, insbesondere zum Färben keratinischer Fasern, der Struktur (I),
X - S - Y (I)
in der X steht für eine Gruppe, die abgeleitet ist von einem direktziehenden Farbstoff,
Y steht für eine Gruppe, die abgeleitet ist von
- einem Oxidationsfarbstoffvorprodukt vom Kuppler- oder Entwicklertyp oder
- einem Derivat des Indols oder Indolins als Vorläufer des Melanins
S steht für eine direkte Bindung oder eine Spacer-Gruppe

2. Mittel zum Färben keratinischer Fasern, **dadurch gekennzeichnet, daß** es einen Hybridfarbstoff der Struktur (I) enthält,
X - S - Y (I)
in der X steht für eine Gruppe, die abgeleitet ist von einem direktziehenden Farbstoff,
Y steht für eine Gruppe, die abgeleitet ist von
- einem Oxidationsfarbstoffvorpmdukt vom Kuppler- oder Entwicklertyp oder
- einem Derivat des Indols oder Indolins als Vorläufer des Melanins
und
S steht für eine direkte Bindung oder eine Spacer-Gruppe

3. Mittel nach Anspruch 2, **dadurch gekennzeichnet, daß** es weiterhin ein Oxidationsfarbstoffvorprodukt vom Kuppler- oder Entwicklertyp enthält.

4. Mittel nach Anspruch 2 oder 3, **dadurch gekennzeichnet, daß** es weiterhin einen direktziehenden Farbstoff enthält.

5. Mittel nach einem der Ansprüche 2 bis 4, **dadurch gekennzeichnet, daß** es weiterhin ein Derivat des Indols oder Indolins als Vorläufer des Melanins enthält.

6. Mittel nach einem der Ansprüche 2 bis 5, **dadurch gekennzeichnet, daß** es ein Tensid enthält.

7. Mittel nach Anspruch 6, **dadurch gekennzeichnet, daß** es sich um ein anionisches Tensid handelt.

8. Mittel nach einem der Ansprüche 2 bis 7, **dadurch gekennzeichnet, daß** es ein kationisches Polymer oder ein Ampho-Polymer enthält.

9. Mittel nach einem der Ansprüche 2 bis 8, **dadurch gekennzeichnet, daß** es ein anionisches oder nichtionogenes Polymer enthält.

10. Verwendung eines Hybridfarbstoffes der Struktur (I) nach Anspruch 1 zum Färben keratinischer Fasern, insbesondere menschlicher Haare.

11. Verwendung eines Hybridfarbstoffes der Struktur (I) nach Anspruch 1 zum Färben der menschlichen Haut.

## Claims

1. A hybrid dye, more particularly for coloring keratin fibers, which corresponds to formula (I):
X-S-Y (I)
where
X is a group derived from a substantive dye,
Y is a group derived from
- an oxidation dye precursor of the secondary or primary intermediate type,
- a derivative of indole or indoline as a precursor of melanin and
S is a direct bond or a spacer group.

2. A composition for coloring keratin fibers, **characterized in that** it contains a hybrid dye corresponding to formula (I):
X-S-Y (I)
where
X is a group derived from a substantive dye,
Y is a group derived from
- an oxidation dye precursor of the secondary or primary intermediate type,
- a derivative of indole or indoline as a precursor of melanin and
S is a direct bond or a spacer group.

3. A composition as claimed in claim 2, **characterized in that** it additionally contains an oxidation dye precursor of the secondary or primary intermediate type.

4. A composition as claimed in claim 2 or 3, **characterized in that** it additionally contains a substantive dye.

5. A composition as claimed in any of claims 2 to 4, **characterized in that** it additionally contains a derivative of indole or indoline as the melanin precursor.

6. A composition as claimed in any of claims 2 to 5, **characterized in that** it contains a surfactant.

7. A composition as claimed in claim 6, **characterized in that** the surfactant is an anionic surfactant.

8. A composition as claimed in any of claims 2 to 7, **characterized in that** it contains a cationic polymer or an amphopolymer.

9. A composition as claimed in any of claims 2 to 8, **characterized in that** it contains an anionic or nonionic polymer.

10. The use of the hybrid dye of formula (I) claimed in claim 1 for coloring keratin fibers, more particularly human hair.

11. The use of the hybrid dye of formula (I) claimed in claim 1 for coloring human skin.

## Revendications

1. Colorant hybride, en particulier pour colorer des fibres de kératine, de structure (l),
X - S - Y (I)
dans laquelle X représente un groupe, qui est dérivé d'un colorant montant directement sur les fibres,
Y représente un groupe, qui est dérivé
- d'un précurseur de colorant par oxydation du type agent de couplage ou révélateur ou
- d'un dérivé de l'indole ou de l'indoline, en tant que précurseur de la mélanine,
S représente une liaison directe ou un groupe intercalaire.

2. Agent pour colorer des fibres de kératine, **caractérisé en ce qu'**il contient un colorant hybride de structure (l),
X - S - Y (I)
dans laquelle X représente un groupe, qui est dérivé d'un colorant montant directement sur les fibres,
Y représente un groupe, qui est dérivé
- d'un précurseur de colorant par oxydation du type agent de couplage ou révélateur ou
- d'un dérivé de l'indole ou de l'indoline, en tant que précurseur de la mélanine et
S représente une liaison directe ou un groupe intercalaire.

3. Agent selon la revendication 2, **caractérisé en ce qu'**il contient en outre un précurseur de colorant par oxydation, du type agent de couplage ou révélateur.

4. Agent selon la revendication 2 ou 3, **caractérisé en ce qu'**il contient en outre un colorant montant directement sur les fibres.

5. Agent selon l'une quelconque des revendications 2 à 4, **caractérisé en ce qu'**il contient en outre un dérivé de l'indole ou de l'indoline, en tant que précurseur de la mélanine.

6. Agent selon l'une quelconque des revendications 2 à 5, **caractérisé en ce qu'**il contient un tensio-actif.

7. Agent selon la revendication 6, **caractérisé en ce qu'**il s'agit d'un tensio-actif anionique.

8. Agent selon l'une quelconque des revendications 2 à 7, **caractérisé en ce qu'**il contient un polymère cationique ou un polymère amphotère.

9. Agent selon l'une quelconque des revendications 2 à 8, **caractérisé en ce qu'**il contient un polymère anionique ou non ionogène.

10. Utilisation d'un colorant hybride de structure (l) selon la revendication 1, pour colorer les fibres de kératine, en particulier les cheveux humains.

11. Utilisation d'un colorant hybride de structure (l) selon la revendication 1, pour colorer la peau humaine.
